# EUROPEAN PATENT SPECIFICATION

(11) **EP 1 981 586 B1**
(45) Date of publication and mention of the grant of the patent: **19.08.2015**
(21) Application number: 06704585.6
(22) Date of filing: 31.01.2006
(51) Int. Cl.: A61N 1/368, A61N 1/362

(54) **SYSTEM FOR DETECTING ELECTRIC EVENTS IN CHAMBERS OF A HEART**
SYSTEM ZUR ERFASSUNG ELEKTRISCHER VORKOMMNISSE IN HERZKAMMERN
SYSTÈME DE DÉTECTION D'ÉVÉNEMENTS ÉLECTRIQUES DANS DES CAVITÉS CARDIAQUES

(43) Date of publication of application: 22.10.2008
(73) Proprietor: St. Jude Medical AB, 175 84 Järfälla (SE)
(72) Inventor: HOLMSTRÖM, Nils, 175 57 Järfälla (SE); BJÖRLING, Anders, 169 37 Solna (SE)
(74) Representative: Sjölander, Henrik
(86) International application number: PCT/SE2006/000143
(87) International publication number: WO 2007/089176

(56) References cited:
- US-A- 5 800 465
- US-A- 5 871 507
- US-A- 5 902 324
- US-A1- 2001 049 543
- US-A1- 2003 204 212
- US-A1- 2004 064 159
- US-A1- 2004 082 975
- US-A1- 2004 172 065
- US-A1- 2004 193 225
- US-A1- 2005 137 638
- US-B1- 6 421 564
- US-B1- 6 493 584
- US-B1- 6 885 893

## Description

### Technical Field

The present invention relates to a system for detecting electric cardiac events in chambers of a heart comprising leads with electrodes adapted for implantation in at least two different chambers of said heart for sensing electric signals therein, and a difference forming means provided to supply a difference signal, formed of said signals sensed by said electrodes, to an event detector, arranged to distinguish, from said difference signal, between events originating from one of said chambers from events originating from the other one of said chambers. The invention also relates to a corresponding heart stimulator comprising the mentioned system.

### Background

Multi-chamber sensing system for use in e.g. multi-chamber pacing systems, need to sense electrical cardiac events, like P and R-waves, at several positions inside or outside of the heart muscle. Leads provided with one or more electrodes are used for this purpose.

Bipolar sensing of both atrial and ventricular events is thus described in US 5 800 468.

Differential sensing between unipolar electrode leads located in the right atrium and the right ventricle is disclosed in US 5 871 507.

In US 2001/0049543 biventricular stimulation and capture monitoring are described. For capture detection cross-chamber sensing electrode configurations are used. Multipolar leads are used and different electrodes are used for stimulation and sensing. Sensing between a right ventricular ring electrode and a left atrial ring electrode is described as well as between right and left ventricular ring electrodes.

To be able to position electrode leads in the coronary veins for stimulation and sensing on the left ventricle and atrium of as many patients as possible with varying anatomy, it is important that the leads are as thin and flexible as possible. Leads with at least an unipolar distal end portion are easier to make thin and flexible and are therefore normally easier to implant in the coronary veins. However, when using an unipolar electrode for sensing with the stimulator or sensing apparatus case as indifferent electrode, the signal-to-noise ratio often becomes unacceptable low.

US 2004/0082975 discloses determination of capture in multiple chambers of a patient's heart using an electrode inserted into a coronary vein. Cardiac signals responsive to pace pulses, applied to multiple heart chambers simultaneously or according to a phased timing sequences, sensed using the coronary vein electrode may be used to verify capture in the multiple chambers of the heart.

The purpose of the present invention is to solve this problem and make detection of capture and loss of capture possible in all the chambers of the heart, also of the left atrium and left ventricle without using the above-mentioned conventional unipolar sensing which often results in a too low signal-to-noise ratio as mentioned.

### Disclosure of the Invention

This purpose is obtained by a system of the kind defined in the introductory portion which have the characterizing features of claim 1.

Thus, according to the present invention detection of electric cardiac events is possible in any one of the four chambers of the heart while avoiding the disadvantage of the above discussed, previously used technique for unipolar sensing by performing the sensing between electrodes located within or on the heart.

According to an advantageous embodiment of the system according to the invention said leads adapted for implantation in a coronary vein on the left side of the heart are unipolar. The use of unipolar left side leads involves several important advantages. Unipolar leads are thinner than bipolar leads, they are softer and more flexible at the distal end portion, have a simpler mechanical construction and a better longevity. An unipolar lead can be more easily bent distally using a pre-bent or steerable stylet which is important for making passage of sharp bends in coronary vessels possible. It is also easier to slide a unipolar lead over a sharply bent guidewire inside a coronary vein and to retract the guidewire after positioning the lead tip in a proper position. Bipolar leads, on the other hand, are often too thick for implantation in all the coronary veins which can be candidates for e.g. left ventricular stimulation and sensing. It can for instance be impossible to pass a sharp bend because the distal end of the bipolar lead is too stiff. The advantages of bipolar leads are that they normally give a higher signal-to-noise ratio, and bipolar stimulation reduces the risk of muscular stimulation at the stimulator case. Bipolar sensing can, however, sometimes be difficult when an action potential is passing both electrodes simultaneously and the signals are similar. In such a case unipolar sensing is preferred. Phrenicus stimulation also often requires attention at left ventricular pacing, since bipolar pacing on the left ventricle increases the risk of phrenicus stimulation because both the tip and the ring are involved. With this embodiment of the invention the risk of phrenicus stimulation is reduced compared to normal bipolar or unipolar stimulation which also can result in pectoral muscle stimulation when higher stimulation amplitudes are needed. To sum up, with the present embodiment the physician will be more free to find an optimal location of the implanted lead on the left side of the heart, which can result in a higher phrenicus stimulation threshold, better hemodynamics and low stimulation threshold, while not loosing the good properties of using bipolar leads.

When stimulating between tip electrodes located in the left and right ventricles it is difficult to detect evoked response between the tips, because the difference signal can be small or totally disappear in case of capture on both the ventricles. In this case a detection can be erroneously interpreted as loss of capture in both ventricles instead of capture in both ventricles. According to other advantageous embodiments of the system according to the invention said event detector is therefore arranged to distinguish, after bi-ventricular stimulation, between capture and non-capture from said difference signal, formed of signals sensed by an electrode positioned in the left ventricle and by a differently positioned electrode on the right side of the heart, said differently positioned electrode on the right side of the heart preferably being a ring or coil electrode. In this way there will be a time lag between evoked response signals sensed in the right and left side of the heart depending on the distance between the ventricular tip electrode and said differently positioned electrode on the right side of the heart and on the propagation velocity of the action potential in the myocardium, when pacing takes place simultaneously on both sides of the heart and capture of both ventricles is obtained.

According to still another advantageous embodiment of the system according to the invention said electrodes are adapted for implantation in a coronary vein on the left atrium, and in a coronary vein on the left ventricle or in the right ventricle respectively. Sensing between a tip electrode in the left atrium and a tip electrode in the left ventricle can be an attractive alternative. It is possible to distinguish between a P-wave and a R-wave from the direction of the rapid voltage change when the action potential passes the electrodes. By using an electrode in one of the ventricles as indifferent electrode when sensing P-waves in the left atrium interference of P-waves in the right atrium is avoided.

### Brief Description of the Drawings

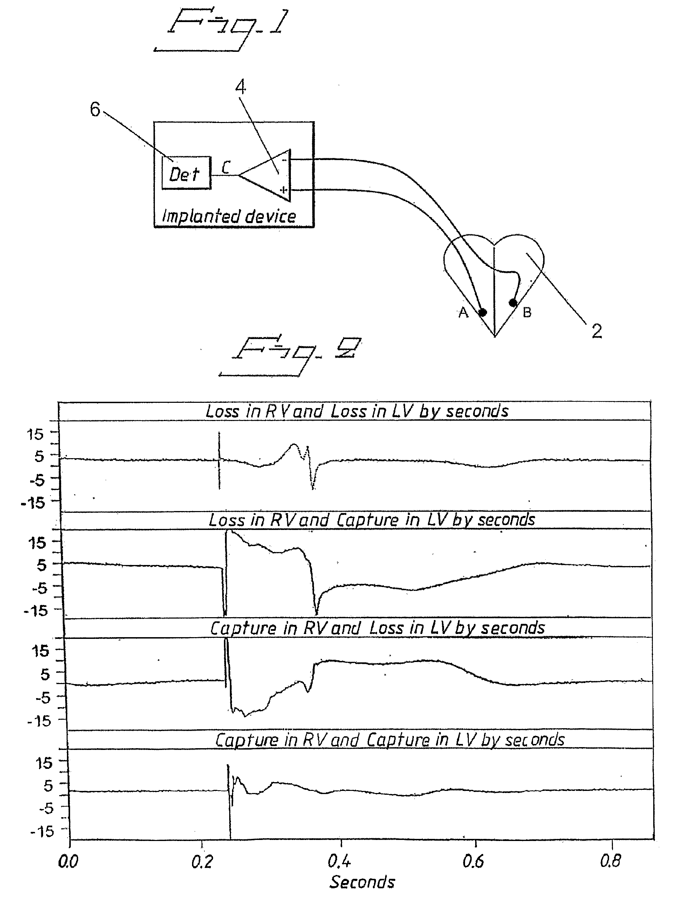

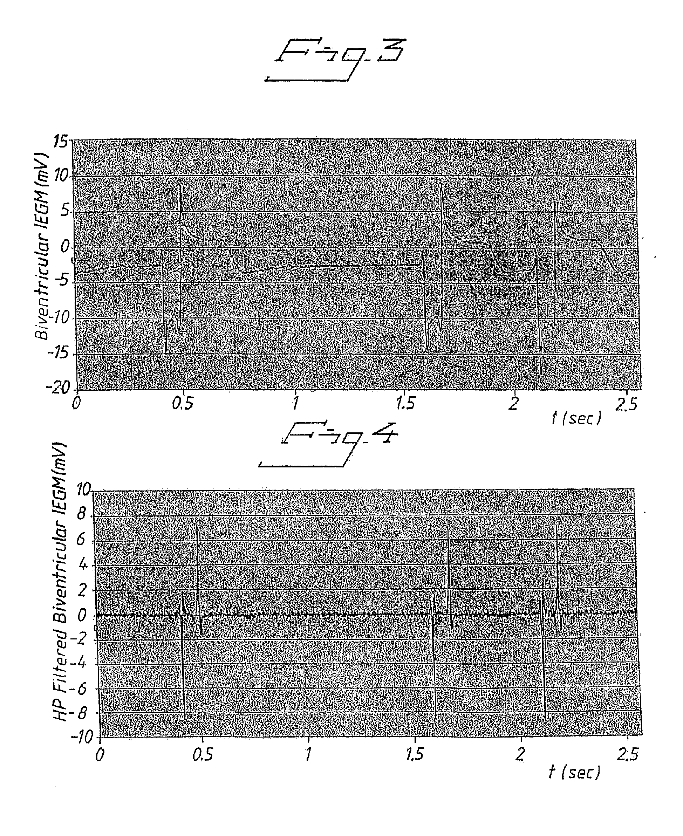

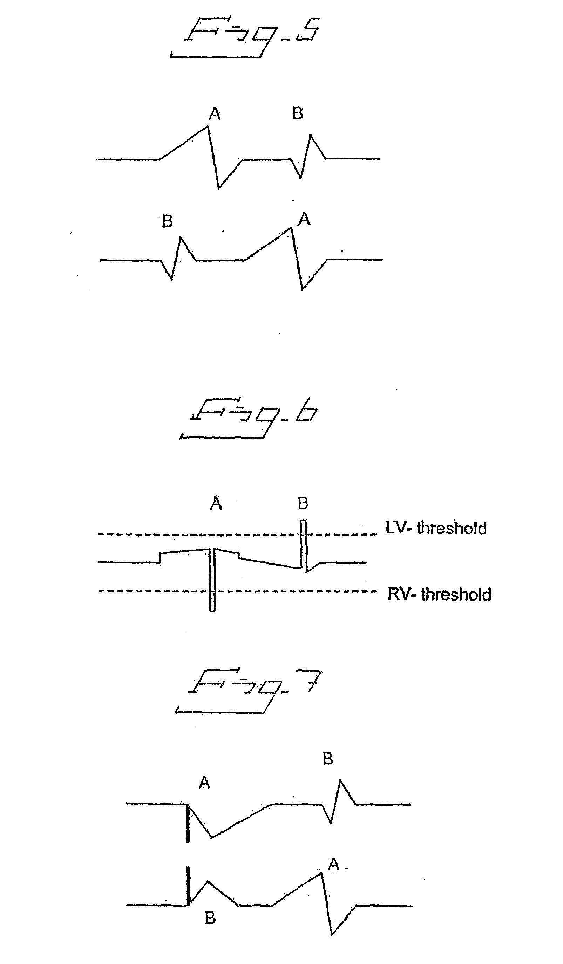

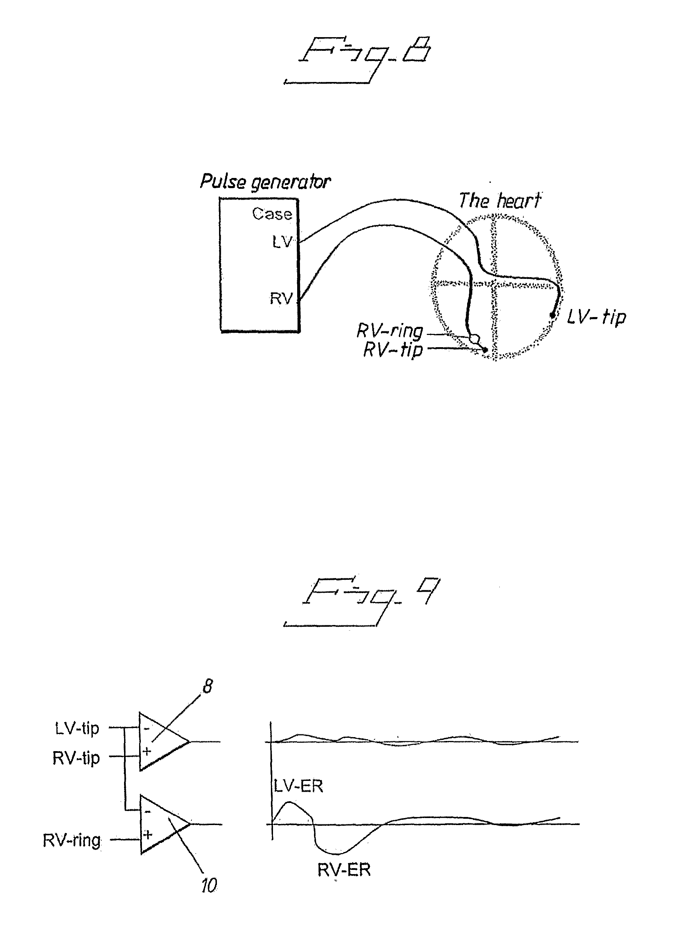

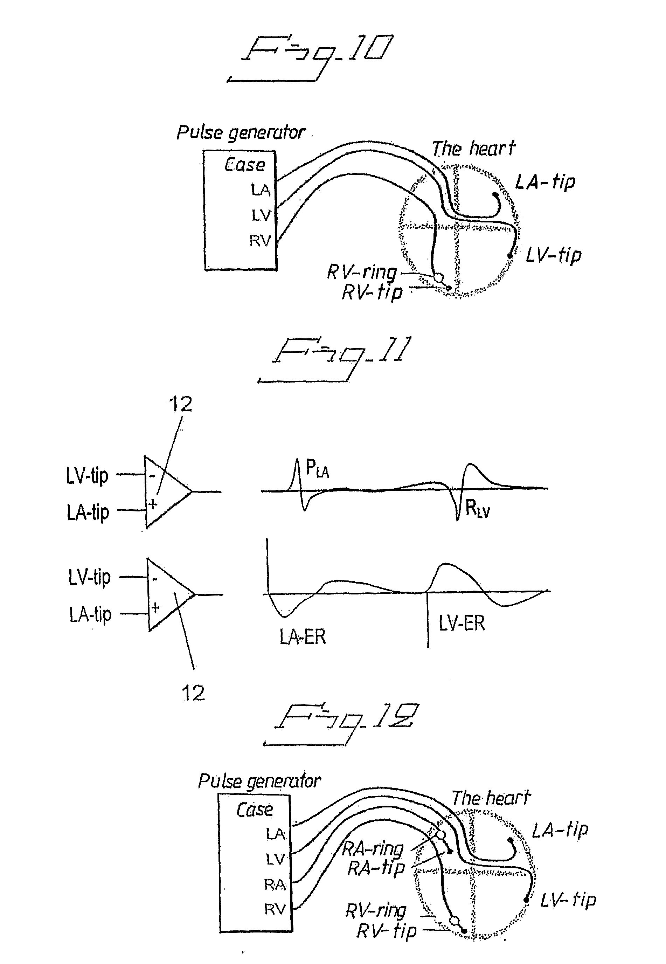

The invention will be described in greater detail with reference to the accompanying drawings, on which figure 1 schematically illustrates a bi-ventricular pacemaker system with bi-ventricular sensing, figure 2 shows intracardiac electrograms, IEGM, between right and left ventricular electrode tips recorded in heart failure patients, figure 3 an IEGM measured between right and left ventricular electrode tips for a heart failure patient having Left Bundle Branch Block, LBBB, figure 4 shows the IEGM signal in figure 3 differentiated, figure 5 shows principally two examples of the signal sensed between the right and left ventricular electrode tips and figure 6 principally the derivative of the signal according to one of the examples in figure 5, figure 7 shows simplified curves representing expected responses after pacing, figure 8 shows schematically an embodiment using a bipolar electrode in the right ventricle, RV, and a unipolar electrode on the left ventricle, LV, and figure 9 illustrates schematically an associated detector and evoked response signals in case of capture of both ventricles, figure 10 shows schematically an embodiment also comprising an additional unipolar electrode lead implanted in a coronary vein on the left atrium, LA, and figure 11 the difference signal sensed between the electrodes on the left side of the heart, and figure 12 illustrates schematically a four-chamber pacemaker system having unipolar leads on the left side of the heart and bipolar electrode leads on the right side.

### Detailed Description of Embodiments of the Invention.

Figure 1 shows schematically an implanted device comprising a bi-ventricular pacemaker system having bi-ventricular sensing. One electrode A is implanted in the RV of a heart 2 and another electrode B is implanted in a coronary vein on the LV. The sensed difference signal C obtained as output signal from the difference forming means 4 is supplied to an event detector 6 for determining both origin and time of electric cardiac events.

Figure 2 shows IEGMs recorded between tip electrodes in RV and LV, cf. figure 1, in heart failure patients having LBBB after bi-ventricular pacing between the tip electrodes.

The curve at the bottom of figure 2 shows that capture in both ventricles results in a very small IEGM signal because the evoked responses practically eliminate each other since the signal amplitudes are about the same. The measured RV evoked response signal amplitude amounted to 13.1 mV and the LV evoked response signal amplitude to 14.2 mV.

The second curve from below in figure 2 illustrates a situation with capture in RV and loss of capture in LV. Due to the loss of capture in LV an intrinsic R-wave is generated after about 120 msec.

The second curve from the top of figure 2 illustrates a situation with capture in LV and loss of capture in RV. An intrinsic right ventricular R-wave appears after about 120 msec.

The curve at the top of figure 2 illustrates a situation with loss of capture in both RV and LV. The two intrinsic R-waves appear almost simultaneously.

From figure 2 appears that with these measurements it is possible to distinguish the four situations illustrated from each other. A predetermined negative threshold value is applied in a time window after the pacing pulse for comparison with the sensed IEGM signal for indicating capture in RV and loss of capture in LV. Similarly, a predetermined positive threshold value is applied in a time window after the pacing pulse for comparison with the sensed IEGM signal for indicating capture in LV and loss of capture in RV. Detection of no signal passage of neither the positive nor the negative threshold value means capture of both ventricles or loss of capture for both ventricles.

One way of distinguishing between capture of both ventricles and loss of capture for both ventricles is to perform a separate R-wave detection. In US 6 148 234 an R-wave detector is described which is enabled from the delivery of a stimulation pulse to the end of the heart refractory period, a period of normally about 300 msec. If R-waves are detected in this period there has been loss of capture in one of the ventricles.

Another way of distinguishing capture of both ventricles from loss of capture for both ventricles is to study separately the signals from RV and LV respectively in case of detection of no difference signal.

Still other possibilities of distinguishing capture of both ventricles from loss of capture for both ventricles will be described below.

Sensing between tip electrodes in RV and LV gives almost a bipolar suppression of noise signals. In figure 3 the intrinsic tip to tip IEGM signal from a heart failure patient having LBBB is shown. The QRS consists of a fast negative deflection signal relating to RV. After about 80 msec a fast positive signal deflection appears relating to LV.

By high pass filtering or differentiating the signal in figure 3 a curve as shown in figure 4 is obtained representing the derivative of the signal in figure 3. Negative peaks in the derivated signal corresponds to R-waves in RV and positive peaks to R-waves in LV. A threshold detector can then be easily used for detecting the R-waves.

Figure 5 shows a simplified representation of difference signals sensed between the ventricular tip electrodes A and B in figure 1. The upper curve in figure 5 shows that the R-wave in RV appears earlier than the R-wave in LV. The lower curve illustrates a situation in which the R-wave in LV comes first.

As described above a suitable high pass filter can be used for making it easier to distinguish between different cardiac events. High pass filtering will enhance the fast derivatives of the IEGM, cf. the discussion above of figures 3 and 4. This is illustrated in figure 6 which shows in a simplified way the derivative dC/dt of the difference signal C. This curve clearly reveals the origins of the cardiac events and detection of them is easily carried out by comparisons with LV- and RV-thresholds.

Another technique for distinguishing between cardiac events originating from one of said chambers from events originating from the other one comprises morphology analysis of the difference signal. When the action potential passes the electrode the potential is first raised, thereafter a fast negative slope occurs followed by a return to the baseline. Depending on which (positive or negative) inputs of the difference forming means 4 the electrodes A and B are connected to, see figure 1, the difference signal C is inverted or not, cf. figure 5.

Figure 7 shows simplified graphs of evoked response signals after capture in case of bi-ventricular stimulation. The upper graph illustrates capture in RV and an (inverted) R-wave in LV, and the bottom graph (inverted) capture in LV and an R-wave in RV. Thus, cardiac events originating from one of the heart chambers can also be distinguished from events originating from the other chamber by analysis of the polarity of the difference signal. Alternatively the cardiac event detector can comprise integrating means for time integrating the difference signal in a predetermined time window to distinguish between events originating from one of the chambers from events originating from the other one of the chambers by analysis of the integrated difference signal value.

In this connection it could be noted that bi-ventricular pacing between the tip electrodes in RV and LV can create capture in both ventricles simultaneously by one single pacing pulse. Normally the anodic stimulation threshold is higher than the cathodic one. Therefore it is possible to change pacing polarity so that the electrode with the higher stimulation threshold is stimulated cathodically (negatively) and the electrode with the lower stimulation threshold - normally the tip electrode in RV - is stimulated anodically (positively). The transventricular (right ventricular tip electrode to left ventricular tip electrode) stimulation threshold will then be lower. Transventricular pacing will also be associated with a low risk of creating phrenicus nerve stimulation, because the pacing current flows through the heart and not to the pacemaker case or along the lead.

As explained above it is preferred to use unipolar electrodes on the left side of the heart. It is well known to use unipolar as well as bipolar electrodes in the right side of the heart. In this connection it could be an advantage to use bipolar electrodes in right atrium and right ventricle.

Above sensing of evoked response between tip electrodes in RV and LV respectively when pacing between the two tip electrodes has been described. As explained above, when detecting evoked response, ER, between ventricular electrode tips in this way the difference signal can be very small or totally disappear when having capture on both ventricles or loss of capture on both ventricles, see figure 9 upper curve, which shows the difference signal in case of capture on both ventricles. To the left in figure 9 difference forming means in the form of differential detectors 8, 10 are shown. In this case the detection can erroneously be misinterpreted as having loss on both tip electrodes. A way of eliminating this drawback consists in sensing ER between the electrode tip on LV and a differently positioned electrode on the right side of the heart. Figure 8 thus shows a stimulation pulse generator connected to a patient's heart by a biventricular electrode having a ring electrode, RV-ring, and a tip electrode, RV-tip, in RV and an unipolar electrode, LV-tip, in a coronary vein on the LV. In this embodiment ER is sensed between RV-ring and LV-tip. There will then be a time lag between the occurrence of the ER signal on the electrodes of about 10-30 msec, depending on the distance between tip and ring and the propagation velocity of the action potential in the myocardium, when a single differential pacing pulse supplied by the tip electrodes is used for capturing both ventricles. This is illustrated by the lower graph in figure 9. Figure 9 also shows how the electrode leads of figure 8 are connected to the differential detectors 8 and 10.

Figure 10 shows schematically a left atrial lead implanted in a coronary vein on the LA of the heart. The left atrial unipolar lead is suitably implanted in a coronary vein with an entrance from the coronary sinus called "oblique vein of the left atrium". In this example a stimulator pulse generator is connected to a heart by a bipolar lead in RV and unipolar leads implanted in coronary veins on the left atrium, LA-tip, and ventricle, LV-tip. In this setup sensing the difference signal between LA-tip and LV-tip is suitable.

In the left part of figure 11 it is shown how the electrodes LV-tip and LA-tip are connected to a differential detector 12. A P-wave P_{LA} can be distinguished from an R-wave R_{LV} by the direction of the rapid voltage change, which occurs when the action potential passes the electrodes, see the upper graph in figure 11. The lower graph in figure 11 illustrates sensed left atrial ER signal and left ventricular ER signal following stimulation. Left atrial pacing should be made between the LA-tip electrode and a ring or coil electrode in the heart to reduce the risk for phrenicus stimulation, which can occur in case of unipolar stimulation with the pulse generator case used as indifferent electrode. Differential stimulation between the electrodes LA-tip and LV-tip on the left side of the heart should be avoided.

Figure 12 shows an example of four chamber pacing. A pacemaker pulse generator is connected to a heart by thin unipolar leads with an LA-tip electrode and an LV-tip electrode implanted in coronary veins on the left side of the heart, and by bipolar leads with RA-tip electrode, RA-ring electrode, RV-tip electrode and RV-ring electrode in the right side of the heart. Left atrial pacing is preferably applied between the electrodes LA-tip and RA-tip or RV-ring, and left ventricular pacing between the electrodes LV-tip and RV-ring or RA-ring. There are several possibilities for sensing P-waves in the left atrium. In the preferred alternative an electrode in one of the ventricles is used as indifferent electrode in order to avoid interference of P-waves from the right atrium. Similarly for sensing R-waves in LV an atrial electrode is preferably used as indifferent electrode.

## Claims

1. A system for detecting electric cardiac events in chambers of a heart (2) comprising:
a lead with an electrode (B; LV-tip) adapted for implantation in a coronary vein on the left side of said heart (2) for sensing an electric signals in a left ventricle of said heart, and a lead with at least one electrode (A; RV-ring, RV-tip) adapted for implantation in a right ventricle of said heart for sensing an electric signal therein,
a difference forming means (4; 8, 10) provided to supply a difference signal to an event detector (6), and
said event detector (6) arranged to distinguish, after bi-ventricular stimulation and from said difference signal, between capture and non-capture in said left ventricle and from capture and non-capture in said right ventricle, **characterized in that** said difference forming means (4; 8, 10) is arranged to form said difference signal sensed between said electrode (B; LV-tip) adapted for implantation in said coronary vein and said electrode (A; RV-ring, RV-tip) adapted for implantation in said right ventricle.

2. The system according to claim 1, **characterized in that** said lead adapted for implantation in said coronary vein on the left side of said heart (2) is unipolar.

3. The system according to claim 1 or 2, **characterized in that** said event detector (6) is arranged to distinguish, after bi-ventricular stimulation and from said difference signal, between four situations, capture in said left ventricle and loss of capture in said right ventricle, loss of capture in said left ventricle and capture in said right ventricle, capture in said left ventricle and capture in said right ventricle, and loss of capture in said left ventricle and loss of capture in said right ventricle.

4. The system according to any of the preceding claims, **characterized in that** said event detector (6) is arranged to distinguish, after bi-ventricular stimulation applied between said left ventricular tip electrode (LV-tip) and said right ventricular tip electrode (RV-tip), between capture and non-capture in said left ventricle and from capture and non-capture in said right ventricle from said difference signal, formed of signals sensed between said left ventricular tip electrode and a right ventricular ring electrode or a coil electrode.

5. The system according to any one of the preceding claims, **characterized in that** said event detector (6) is arranged to distinguish between events originating from said left ventricle from events originating from said right ventricle by morphology analysis of said difference signal.

6. The system according to any one of the claims 1 - 4, **characterized in that** said event detector (6) is arranged to distinguish between events originating from said left ventricle from events originating from said right ventricle by analysis of polarity of said difference signal.

7. The system according to any one of the preceding claims, **characterized in that** said event detector (6) comprises integrating means for time integrating said difference signal in a predetermined time window to distinguish between events originating from said left ventricle from events originating from said right ventricle by analysis of the integrated difference signal value.

8. The system according to any one of the preceding claims, **characterized in that** said event detector (6) comprises a high-pass filter or a differentiating means for enhancing derivatives of said difference signal, and **in that** a comparator is provided to compare said derivatives with pre-set threshold values for distinguishing events originating from said left ventricle from events originating from said right ventricle.

9. The system according to any one of the preceding claims, **characterized in that** said event detector (6) is arranged to determine origin and time of the occurrence of said electric events.

10. An implantable heart stimulator, **characterized in that** it comprises a system according to any one of the preceding claims.

11. The heart stimulator according to claim 10, **characterized in that** a separate R-wave detector is connected to said event detector (6), said R-wave detector being enabled from the delivery of a stimulation pulse to the end of the heart refractory period, and **in that** said event detector (6) is arranged to distinguish, from the output signal from said R-wave detector, loss in both ventricles from capture in both ventricles.

## Patentansprüche

1. System zur Erfassung elektrischer kardialer Ereignisse in Kammern eines Herzens (2), umfassend:
eine Leitung mit einer Elektrode (B; LV-tip), die zur Implantation in einer Koronarvene auf der linken Seite des Herzens (2) zum Erfassen eines elektrischen Signals in einer linken Herzkammer des Herzens ausgelegt ist, und eine Leitung mit mindestens einer Elektrode (A; RV-ring, RV-tip), die zur Implantation in einer rechten Herzkammer des Herzens zum Erfassen eines elektrischen Signals darin ausgelegt ist,
ein differenzbildendes Mittel (4; 8, 10), das dafür vorgesehen ist, ein Differenzsignal an einen Ereignisdetektor (6) zu liefern, und
wobei der Ereignisdetektor (6) so angeordnet ist, dass er, nach einer biventrikulären Stimulation und anhand des Differenzsignals, zwischen Reizantwort und keiner Reizantwort in der linken Herzkammer und von Reizantwort und keiner Reizantwort in der rechten Herzkammer unterscheidet, **dadurch gekennzeichnet, dass** das differenzbildende Mittel (4; 8, 10) so angeordnet ist, dass es das Differenzsignal bildet, das zwischen der Elektrode (B; LV-tip), die zur Implantation in der Koronarvene ausgelegt ist, und der Elektrode (A; RV-ring, RV-tip) erfasst wird, die zur Implantation in der rechten Herzkammer ausgelegt ist.

2. System nach Anspruch 1, **dadurch gekennzeichnet, dass** die Leitung, die zur Implantation in der Koronarvene auf der linken Seite des Herzens (2) ausgelegt ist, unipolar ist.

3. System nach Anspruch 1 oder 2, **dadurch gekennzeichnet, dass** der Ereignisdetektor (6) so angeordnet ist, dass er, nach einer biventrikulären Stimulation und anhand des Differenzsignals, zwischen vier Situationen unterscheidet, Reizantwort in der linken Herzkammer und Verlust der Reizantwort in der rechten Herzkammer, Verlust der Reizantwort in der linken Herzkammer und Reizantwort in der rechten Herzkammer, Reizantwort in der linken Herzkammer und Reizantwort in der rechten Herzkammer, und Verlust der Reizantwort in der linken Herzkammer und Verlust der Reizantwort in der rechten Herzkammer.

4. System nach einem der vorhergehenden Ansprüche, **dadurch gekennzeichnet, dass** der Ereignisdetektor (6) so angeordnet ist, dass er, nach einer biventrikulären Stimulation, die zwischen der Spitzenelektrode der linken Herzkammer (LV-tip) und der Spitzenelektrode der rechten Herzkammer (RV-tip) angelegt wird, zwischen Reizantwort und keiner Reizantwort in der linken Herzkammer und von Reizantwort und keiner Reizantwort in der rechten Herzkammer anhand des Differenzsignals unterscheidet, das aus Signalen gebildet wird, die zwischen der Spitzenelektrode der linken Herzkammer und einer Ringelektrode oder Wendelelektrode der rechten Herzkammer erfasst werden.

5. System nach einem der vorhergehenden Ansprüche, **dadurch gekennzeichnet, dass** der Ereignisdetektor (6) so angeordnet ist, dass er zwischen Ereignissen, die von der linken Herzkammer ausgehen, von Ereignissen, die von der rechten Herzkammer ausgehen, mittels Morphologieanalyse des Differenzsignals unterscheidet.

6. System nach einem der Ansprüche 1 bis 4, **dadurch gekennzeichnet, dass** der Ereignisdetektor (6) so angeordnet ist, dass er zwischen Ereignissen, die von der linken Herzkammer ausgehen, von Ereignissen, die von der rechten Herzkammer ausgehen, mittels Polaritätsanalyse des Differenzsignals unterscheidet.

7. System nach einem der vorhergehenden Ansprüche, **dadurch gekennzeichnet, dass** der Ereignisdetektor (6) Integrationsmittel für die Zeitintegration des Differenzsignals in einem vorher festgelegten Zeitfenster umfasst, damit er zwischen Ereignissen, die von der linken Herzkammer ausgehen, von Ereignissen, die von der rechten Herzkammer ausgehen, mittels Analyse des integrierten Differenzsignalwerts unterscheidet.

8. System nach einem der vorhergehenden Ansprüche, **dadurch gekennzeichnet, dass** der Ereignisdetektor (6) ein Hochpassfilter oder ein Differenziermittel zum Verstärken von Ableitungen des Differenzsignals umfasst, und dadurch, dass eine Vergleichseinrichtung zum Vergleichen der Ableitungen mit voreingestellten Schwellenwerten vorgesehen ist, damit Ereignisse, die von der linken Herzkammer ausgehen, von Ereignissen, die von der rechten Herzkammer ausgehen, unterschieden werden.

9. System nach einem der vorhergehenden Ansprüche, **dadurch gekennzeichnet, dass** der Ereignisdetektor (6) so angeordnet ist, dass er Ursprung und Zeit des Auftretens der elektrischen Ereignisse bestimmt.

10. Implantierbarer Herzstimulator, **dadurch gekennzeichnet, dass** er ein System nach einem der vorhergehenden Ansprüche umfasst.

11. Herzstimulator nach Anspruch 10, **dadurch gekennzeichnet, dass** ein separater R-Wellen-Detektor an den Ereignisdetektor (6) angeschlossen ist, wobei der R-Wellen-Detektor von der Abgabe eines Stimulationsimpulses bis zum Ende der Refraktärzeit des Herzens aktiviert ist, und dadurch, dass der Ereignisdetektor (6) so angeordnet ist, dass er, anhand des Ausgangssignals von dem R-Wellen-Detektor, einen Verlust in beiden Herzkammern von einer Reizantwort in beiden Herzkammern unterscheidet.

## Revendications

1. Système de détection d'événements électriques cardiaques dans des cavités d'un coeur (2) comprenant :
une dérivation avec une électrode (B ; LV-tip) adaptée pour l'implantation dans une veine coronaire sur le côté gauche dudit coeur (2) afin de détecter un signal électrique dans un ventricule gauche dudit coeur, et une dérivation avec au moins une électrode (A ; RV-ring, RV-tip) adaptée pour l'implantation dans un ventricule droit dudit coeur afin de détecter un signal électrique dans celui-ci,
un moyen de formation de différence (4 ; 8, 10) destiné à fournir un signal de différence à un détecteur d'événements (6), et
ledit détecteur d'événements (6) étant agencé pour faire une distinction, après une stimulation biventriculaire et à partir dudit signal de différence, entre capture et absence de capture dans ledit ventricule gauche et capture et absence de capture dans ledit ventricule droit, **caractérisé en ce que** ledit moyen de formation de différence (4 ; 8, 10) est agencé pour former ledit signal de différence détecté entre ladite électrode (B ; LV-tip) adaptée pour l'implantation dans ladite veine coronaire et ladite électrode (A ; RV-ring, RV-tip) adaptée pour l'implantation dans ledit ventricule droit.

2. Système selon la revendication 1, **caractérisé en ce que** ladite dérivation adaptée pour l'implantation dans ladite veine coronaire sur le côté gauche dudit coeur (2) est unipolaire.

3. Système selon la revendication 1 ou 2, **caractérisé en ce que** ledit détecteur d'événements (6) est agencé pour faire la distinction, après une stimulation biventriculaire et à partir dudit signal de différence, entre quatre situations, capture dans ledit ventricule gauche et perte de capture dans ledit ventricule droit, perte de capture dans ledit ventricule gauche et capture dans ledit ventricule droit, capture dans ledit ventricule gauche et capture dans ledit ventricule droit, et perte de capture dans ledit ventricule gauche et perte de capture dans ledit ventricule droit.

4. Système selon l'une quelconque des revendications précédentes, **caractérisé en ce que** ledit détecteur d'événements (6) est agencé pour faire la distinction, après une stimulation biventriculaire appliquée entre ladite électrode pointue du ventricule gauche (LV-tip) et ladite électrode pointue du ventricule droit (RV-tip), entre capture et absence de capture dans ledit ventricule gauche et capture et absence de capture dans ledit ventricule droit à partir dudit signal de différence, formé à partir de signaux détectés entre ladite électrode pointue du ventricule gauche et une électrode annulaire ou une électrode hélicoïdale du ventricule droit.

5. Système selon l'une quelconque des revendications précédentes, **caractérisé en ce que** ledit détecteur d'événements (6) est agencé pour faire la distinction entre des événements provenant dudit ventricule gauche et des événements provenant dudit ventricule droit par analyse morphologique dudit signal de différence.

6. Système selon l'une quelconque des revendications 1 à 4, **caractérisé en ce que** ledit détecteur d'événements (6) est agencé pour faire la distinction entre des événements provenant dudit ventricule gauche et des événements provenant dudit ventricule droit par analyse de polarité dudit signal de différence.

7. Système selon l'une quelconque des revendications précédentes, **caractérisé en ce que** ledit détecteur d'événements (6) comprend des moyens d'intégration destinés à intégrer dans le temps ledit signal de différence dans une fenêtre temporelle prédéterminée pour faire la distinction entre des événements provenant dudit ventricule gauche et des événements provenant dudit ventricule droit par analyse de la valeur du signal de différence intégrée.

8. Système selon l'une quelconque des revendications précédentes, **caractérisé en ce que** ledit détecteur d'événements (6) comprend un filtre passe-haut ou un moyen de différentiation destiné à amplifier des dérivées dudit signal de différence, et **en ce qu'**un comparateur est destiné à comparer lesdites dérivées avec des valeurs seuil préréglées pour faire la distinction entre des événements provenant dudit ventricule gauche et des événements provenant dudit ventricule droit.

9. Système selon l'une quelconque des revendications précédentes, **caractérisé en ce que** ledit détecteur d'événements (6) est agencé pour déterminer l'origine et le temps de l'occurrence desdits événements électriques.

10. Stimulateur cardiaque implantable, **caractérisé en ce qu'il** comprend un système selon l'une quelconque des revendications précédentes.

11. Stimulateur cardiaque selon la revendication 10, **caractérisé en ce qu'un** détecteur d'onde R séparé est raccordé audit détecteur d'événements (6), ledit détecteur d'onde R étant activé de l'émission d'une impulsion de stimulation à la fin de la période réfractaire du coeur, et **en ce que** ledit détecteur d'événements (6) est agencé pour faire la distinction, à partir du signal de sortie dudit détecteur d'onde R, entre une perte dans les deux ventricules et une capture dans les deux ventricules.
